# EUROPÄISCHE PATENTSCHRIFT

(11) **EP 2 066 265 B1**
(45) Veröffentlichungstag und Bekanntmachung des Hinweises auf die Patenterteilung: **30.03.2016**
(21) Anmeldenummer: 07818520.4
(22) Anmeldetag: 27.09.2007
(51) Int. Cl.: A61F 2/30, A61B 17/80, A61F 2/36, A61F 2/46

(54) **HÜFTIMPLANTAT**
HIP IMPLANT
IMPLANT DE HANCHE

(30) Priorität: 28.09.2006 DE 202006014950 U
(43) Veröffentlichungstag der Anmeldung: 10.06.2009
(73) Patentinhaber: Brehm, Peter, 91085 Weisendorf (DE)
(72) Erfinder: Brehm, Peter, 91085 Weisendorf (DE)
(74) Vertreter: Grünecker Patent- und Rechtsanwälte PartG mbB
(86) Internationale Anmeldenummer: PCT/EP2007/008437
(87) Internationale Veröffentlichungsnummer: WO 2008/037480

(56) Entgegenhaltungen:
- EP-A- 0 257 359
- EP-A- 0 359 485
- DE-A1- 3 336 005
- US-A- 4 790 854

## Beschreibung

Die vorliegende Erfindung bezieht sich allgemein auf Hüftimplantate, insbesondere auf Hüftimplantate mit proximalem Femurersatz, die bei großem Verlust an Knochensubstanz im Femur (Oberschenkelknochen), z.B. infolge eines Tumors, eingesetzt werden können.

Bei Patienten, die an einem Knochentumor im Bereich des Hüftgelenks leiden, zum Beispiel an einem Osteosarkom, kann es notwendig sein, die vom Tumor befallenen Bereiche operativ zu entfernen. Um das Risiko der Bildung von Metastasen zu verringern, muss dabei oft auch Gewebe in der Umgebung des Tumors mit entfernt werden. Dadurch kann es vorkommen, dass der Patient ein natürliches Hüftgelenk verliert und einen künstlichen Ersatz benötigt, oder dass ein bereits vorhandenes künstliches Hüftgelenk des Patienten ersetzt werden muss.

Auch wenn sich Metastasen eines Tumors eines anderen Organs in einem Knochen in der Umgebung des Hüftgelenks angesiedelt haben, kann es notwendig sein, das natürliche Hüftgelenk des Patienten und/oder ein großes Stück des Femur zu entfernen, um ein weiteres Wachstum der Metastase zu verhindern. Eine Implantation eines künstlichen Hüftgelenks kann auch nach einem Knochenverlust infolge eines Traumas, oder bei einer schweren Knochendeformität, die z.B. durch eine Osteomyelitis verursacht werden kann, notwendig sein.

Eine Hüftgelenksprothese nach dem Stand der Technik ist beispielsweise in der Druckschrift DE 43 20 086 A1 beschrieben. Diese Druckschrift offenbart ein Revisionshüftprothesensystem mit einem in den Femur einschlagbaren Schaft, einem den Schaft verlängemden Verbindungsabschnitt und einem abgewinkelten Halsteil, das zum Aufsetzen einer Gelenkkugel ausgebildet ist. Der Verbindungsabschnitt ist wenigstens teilweise durch eine Mehrzahl von Hülsensegmenten, die als Verlängerungshülsen dienen, gebildet, die über Konusabschnitte ineinander steckbar sind. Die Hülsensegmente weisen eine durchgehende Längsbohrung auf, in die eine bis in den Schaft reichende Sicherungsschraube einsetzbar ist.

Wenn das Revisionshüftprothesensystem einem Patienten implantiert wird, wird der Schaft in den Femur des Patienten eingesetzt. Das Halsteil wird mit einer Gelenkkugel ausgestattet, die in eine am Hüftknochen des Patienten befestigte Gelenkpfanne eingesetzt wird, so dass sich die Gelenkkugel in der Gelenkpfanne drehen kann. Um die Länge der Prothese an die Anatomie des Patienten anzupassen, kann der Operateur während der Operation eines oder mehrere der Hülsensegmente auswählen, die dann mittels der Sicherungsschraube zwischen dem Schaft und dem Halsteil befestigt werden, wobei die Anwesenheit der Hülsensegmente den Abstand zwischen dem distalen Ende des Schafts und dem Halsteil verlängert.

Wird die oben beschriebene Revisionshüftprothese einem Patienten implantiert, der, z.B. infolge einer Tumorerkrankung, ein großes Stück seines Femur verloren hat, kann es vorkommen, dass zwischen dem Verbindungsabschnitt und dem verbliebenen Teil des Femur ein Teil des Schafts freiliegt. Da der Schaft einen geringeren Durchmesser aufweist als der natürliche Femur eines Menschen, kann die zu einer ungünstigen Konstellation durch das zu lockere Weichteilgewebe in der Umgebung des freiliegenden Teils des Schafts führen. Außerdem kann es vorkommen, dass der Schaft durch mechanische Kräfte, die auftreten, wenn sich der Patient bewegt, in den Femur hineingedrückt wird. Das kann besonders dann vorkommen, wenn der Schaft zementfrei in den Femur eingesetzt wurde. Da der Schaft eine leicht konische Form aufweist, treten beim Hineindrücken des Schafts in den Knochen Kräfte auf, die quer zur Längsrichtung des Knochens wirken. Insbesondere bei älteren Patienten, deren Knochen bereits geschwächt sind, beispielsweise durch eine Osteoporose-Erkrankung, können diese Kräfte ausreichen, den Femur zu sprengen.

Um den resezierten Knochenraum aufzufüllen oder ein eventuelles Nachsintem zu vermeiden, wurden spezielle Hüftgelenksprothesen vorgeschlagen, die besonders an die Bedürfnisse von Patienten angepasst wurden, bei denen ein großer Teil des Femur reseziert wurde.

Derartige Prothesen können einen Verankerungsschaft umfassen, der kürzer ist als der Schaft einer gewöhnlichen Hüftgelenksprothese und deshalb vollständig in das verbliebene Stück des Femur des Patienten eingesetzt werden kann. Zwischen dem Schaft und dem Gelenkkugelabschnitt der Prothese wird ein Verlängerungssegment eingesetzt, dessen Durchmesser in etwa dem des natürlichen Femur entspricht. Dadurch kann eine Fixation von Weichteilen in der Umgebung der Prothese erreicht werden. Ein solches System kann auch eingesetzt werden, um ein Hineinrutschen des Schafts in den Knochen zu vermeiden oder das Risiko einer Sprengung des Femur durch Hineinrutschen zu verringern.

Ein Nachteil dieser speziellen Hüftgelenksprothesen ist, dass es sich um vollständige modulare Prothesen handelt, deren Komponenten nicht mit denen anderer Prothesen kombinierbar und austauschbar sind. Deshalb müssen Ärzte die Implantation dieser Prothesen besonders erlernen und können Erfahrungen, die sie mit Standardprothesen erworben haben, nur in eingeschränktem Umfang anwenden. Außerdem müssen beim Hersteller und in medizinischen Einrichtungen, in denen Hüftgelenksprothesen implantiert werden, zusätzlich zu Standardprothesen vollständige Prothesen für Patienten mit großen Oberschenkeldefekten vorrätig gehalten werden. Dadurch entsteht nicht nur ein erhöhter Platzbedarf für die Lagerung der Prothesen, sondern auch ein hoher Kostenfaktor durch die Notwendigkeit ein großes Sortiment an Prothesen zu bevorraten

Ein weiterer Nachteil der Hüftgelenksprothesen zur Versorgung größerer Knochendefekte nach dem Stand der Technik ist, dass anstelle von Standardschäften spezielle, kürzere Schäfte benötigt werden. Derartige Schäfte müssen gesondert konstruiert, hergestellt und gelagert werden, was die Kosten der Hüftgelenksprothese erhöhen kann. Da Schäfte, die sich in Standard-Hüftgelenksprothesen durch ihre Verträglichkeit und Stabilität langjährig bewährt haben, nicht übernommen werden können, erhöht sich das Risiko für den Patienten, der mit einer nur selten verwendeten Spezialkonstruktion leben muss, mit der relativ wenig Erfahrungen hinsichtlich ihrer langfristigen Haltbarkeit im menschlichen Körper gesammelt werden konnten.

Es ist eine Aufgabe der vorliegenden Erfindung, die genannten Nachteile zu vermeiden oder zumindest zu verringern.

Gemäß der vorliegeden Erfindung werden diese Aufgaben durch die Merkmale des Anspruchs 1 gelöst. Das Hüftimplantat umfasstein Zwishenstück mit einem ersten Verbindungselement, das zum Verbinden des Zwischenstücks mit einem Halsteil des Hüftimplantats ausgebildet ist, und ein zweites Verbindungselement, das zum Verbinden des Zwischenstücks mit einem Schaft des Hüftimplantats ausgebildet ist. Das Hüftimplantat umfasst ferner ein Hülsenelement, das eine Längsbohrung aufweist und mit dem Zwischenstück derart verbindbar ist, dass das zweite Verbindungselement durch die Längsbohrung hindurch zugänglich ist.

Das Zwischenstückes kann zwischen dem Schaft und dem Halsteil einer Hüftprothese anstelle eines Zwischenstücks nach dem Stand der Technik, z.B. anstelle des in der Druckschrift DE 43 20 086 A1 beschriebenen Verbindungsabschnitts, eingesetzt werden. Durch die Längsbohrung des Hülsenelements ist das zweite Verbindungselement des Zwischenstücks auch dann zugänglich, wenn das Hülsenelement am Zwischenstück befestigt ist. Der Schaft der Hüftprothese kann deshalb durch die Längsbohrung geführt und in das zweite Verbindungselement eingesetzt werden, so dass das Hülsenelement den Schaft ringförmig umschließt.

Wenn das Hüftimplantat einem Patienten implantiert wird, befindet sich das Hülsenelement zwischen dem verbliebenen Oberschenkelstück des Patienten und dem Halsteil der Hüftprothese. Dadurch kann der Durchmesser des Implantats in diesem Bereich dem des natürlichen menschlichen Femur angepasst werden, wodurch eine Fixation der Weichteile des Patienten verbessert werden kann. Außerdem kann das Hülsenelement ein unerwünschtes Hineinrutschen des Schafts in den Femur verhindern. Somit ermöglicht es das Modul gemäß der vorliegenden Erfindung, eine Hüftprothese an die besonderen Bedürfnisse von Patienten mit großen Femurdefekten anzupassen.

Geeigneterweise umfasst das erste Verbindungselement einen männlichen Konus. Dadurch kann das Zwischenstück in das Halsteil eines Hüftimplantats eingesetzt werden, das einen weiblichen Konus aufweist.

Das zweite Verbindungselement kann einen weiblichen Konus umfassen. In diesen kann ein Schaft eines Hüftimplantats eingesetzt werden.

Geeigneterweise umfasst das Zwischenstück ein Gewinde oder einen Konus und das mindestens eine Hülsenelement weist ein zu dem Gewinde passendes Gegengewinde bzw. einen Gegenkonus auf. Dadurch kann das Hülsenelement an das Zwischenstück angeschraubt und so mit diesem verbunden werden. Die Schraubverbindung kann während der Operation leicht hergestellt werden. Falls es notwendig ist, beispielsweise, um ein Hülsenelement während der Operation durch ein anderes, der Anatomie des Patienten besser angepasstes Hülsenelement zu ersetzen, kann die Schraubverbindung unschwer wieder gelöst werden. Dadurch wird eine flexible Handhabung des Implantats während der Operation und damit eine genaue Anpassung des Implantats an die Anatomie des Patienten ermöglicht.

Das Hüftimplantat kann mehrere Hülsenelemente umfassen, wobei mindestens ein Hülsenelement mit einem anderen Hülsenelement verbindbar ist. Durch Kombination von mehreren Hülsenelementen kann eine Hülse geschaffen werden, die eine Länge hat, die geeignet ist, den Bereich zwischen dem Femur des Patienten und dem Zwischenstück des Moduls optimal abzudecken. Die Länge der Hülse kann dabei an die individuelle Anatomie des Patienten und die Länge des fehlenden Stücks des Femur angepasst werden. Das mindestens eine Hülsenelement kann ein Abschluss-Hülsenelement umfassen, das eine zur Auflage auf einem Femur geeignete Auflagefläche aufweist. Dadurch kann eine gleichmäßige Verteilung von mechanischen Kräften, die von dem mindestens einen Hülsenelement auf den Femur wirken, erreicht werden. So können eine mechanische Beanspruchung des Knochens reduziert und Verletzungen des Knochens verhindert werden.

Vorteilhafterweise weist das Zwischenstück eine Längsbohrung auf, die dafür ausgelegt ist, eine Schraube durchzustecken, um das Halsteil mit dem Schaft zu verschrauben.

Zwischenstück und Hülsenelement können mit einem Hüftimplantat nach dem Stand der Technik kombiniert werden, in dem der Schaft durch einen aus einem oder mehreren Hülsenelementen bestehenden Verbindungsabschnitt mit einem Gelenkkugelabschnitt verbindbar ist, und in dem der Schaft durch eine Sicherungsschraube, die durch die Hülsenelemente geführt wird, mit dem Gelenkkugelabschnitt verschraubt werden kann. Das Zwischenstück ersetzt dabei eine Verlängerungshülse des Implantats nach dem Stand der Technik.

Geeigneterweise weist das mindestens eine Hülsenelement eine Wandung mit einer oder mehreren Öffnungen auf. Dadurch kann eine Bildung abgeschlossener Hohlräume im Inneren des Hüftimplantats vermieden werden. Außerdem können die Öffnungen genutzt werden, um ein Werkzeug zum Verschrauben des mindestens einen Hülsenelements mit dem Zwischenstück anzusetzen.

Vorteilhafterweise umfasst das Hüftimplantat zusätzlich eine Platte, die an dem mindestens einen Hülsenelement und/oder dem Zwischenstück befestigbar ist und mit einem Femur verbindbar ist. Dadurch kann - neben der Verbindung durch den in den Femur eingesetzten Schaft - eine zusätzliche Verbindung zwischen dem Hüftimplantat und dem Femur geschaffen werden. So kann eine Stabilität der Verbindung zwischen dem Hüftimplantat und dem Körper des Patienten verbessert werden.

Durch das Hülsenelement kann ein Bereich zwischen dem Femur des Patienten und dem Zwischenstück, in dem der Schaft des Hüftimplantats aus dem Femur herausragt, abgedeckt werden. Dadurch kann der Durchmesser des Hüftimplantats in diesem Bereich dem Durchmesser des natürlichen menschlichen Femur angepasst werden, wodurch eine verbesserte Fixation von Weichteilen des Patienten erreicht und ein eventuelles Hineinrutschen des Schafts in den Femur vermieden werden kann. Im zusammengebauten Zustand des Implantats umschließt das Hülsenelement, welches das fehlende Stück des Femur ersetzt den Schaft ringförmig und hat damit keinen Einfluss auf den Abstand zwischen dem distalen Ende des Schafts und dem Halsteil. Folglich muss in dem Hüftimplantat gemäß der vorliegenden Erfindung keine Anpassung der Schaftlänge vorgenommen werden, um das Implantat an Patienten anzupassen, denen ein größerer Teil des Femur fehlt. Dadurch kann eine Verwendung eines Standardschafts ermöglicht werden.

Geeigneterweise umfasst der Schaft einen männlichen Konus und umfasst das Zwischenstück einen weiblichen Konus, wobei der weibliche Konus dafür ausgelegt ist, den männlichen Konus aufzunehmen, und wobei eine Achse des weiblichen Konus eine Längsrichtung des Zwischenstücks definiert. Durch die konische Form der Verbindung zwischen dem Schaft und dem Zwischenstück kann eine hohe Stabilität der Verbindung gegenüber mechanischen Belastungen erzielt werden.

Das mindestens eine Hülsenelement kann derart mit dem Zwischenstück verbindbar sein, dass eine Richtung der Längsbohrung des mindestens einen Hülsenelements parallel zur Längsrichtung des Zwischenstücks ist. Dadurch kann die Längsbohrung einen Kanal bilden, der besonders gut zum Durchstecken eines geraden Schafts geeignet ist.

In anderen Ausführungsformen kann das mindestens eine Hülsenelement derart mit dem Zwischenstück verbindbar sein, dass eine Richtung der Längsbohrung des mindestens einen Hülsenelements zur Längsrichtung des Zwischenstücks geneigt ist. Dadurch kann die Längsbohrung einen Kanal bilden, der besonders gut zum Durchstecken eines gebogenen Schafts geeignet ist.

Das Hüftimplantat kann mehrere Hülsenelemente aufweisen, die derart miteinander verbindbar sind, dass der Schaft durch die Längsbohrungen der mehreren Hülsenelemente hindurch in das Zwischenstück einsetzbar ist. Dadurch können die mehreren Hülsenelemente aneinander gesetzt werden, um das Hüftimplantat an Patienten anzupassen, denen ein großes Stück des Femur entfernt wurde.

Geeigneterweise sind die Hülsenelemente derart miteinander verbindbar, dass die Längsbohrungen der Hülsenelemente in die gleiche Richtung verlaufen. Dadurch kann ein gerader Schaft mit geringem Zwischenraum zwischen dem Schaft und den Innenwänden der Längsbohrungen eingesetzt werden. ,

In anderen Ausführungsformen können die Hülsenelemente derart miteinander verbindbar sein, dass die Längsbohrungen von mindestens zweien der Hülsenelemente zueinander geneigt sind. Dadurch kann der Raum im Inneren der Längsbohrungen eine Form erhalten, die besonders gut für die Aufnahme eines gekrümmten Schafts geeignet ist.

Insbesondere kann in dieser Ausführungsform die Achse eines Gewindes eines Hülsenelementes gegen eine Achse ihrer Längsbohrung geneigt sein, um ein verkipptes Anschrauben der anderen Hülsensegmente zu ermöglichen.

Der Schaft kann eine Krümmung aufweisen. Dies kann bei Patienten, deren Femur eine Krümmung aufweist, vorteilhaft sein, um das Implantat an die Anatomie des Patienten anzupassen.

Das Zwischenstück kann einen männlichen Konus aufweisen, der in einen weiblichen Konus des Halsteils einführbar ist. Dadurch kann eine stabile Verbindung zwischen dem Zwischenstück und dem Halsteil geschaffen werden.

Das Zwischenstück und das mindestens eine Hülsenelement können durch Gewinde miteinander verbindbar sein.

Das mindestens eine Hülsenelement kann ein Abschluss-Hülsenelement umfassen, das eine zur Auflage auf einem Femur geeignete Auflagefläche aufweist.

Das Zwischenstück kann eine Längsbohrung aufweisen und das Hüftimplantat kann eine durch die Längsbohrung führbare Schraube umfassen, die dafür geeignet ist, das Halsteil mit dem Schaft zu verschrauben.

Das mindestens eine Hülsenelement kann eine Wandung mit einer oder mehreren Öffnungen aufweisen.

Das Hüftimplantat kann zusätzlich mindestens eine Platte umfassen, die an dem mindestens einen Hülsenelement und/oder dem Zwischenstück befestigbar ist und mit einem Femur verbindbar ist.

Ausführungsformen der vorliegenden Erfindung werden nun mit Bezug auf die beigefügten Zeichnungen beschrieben. Es zeigen:

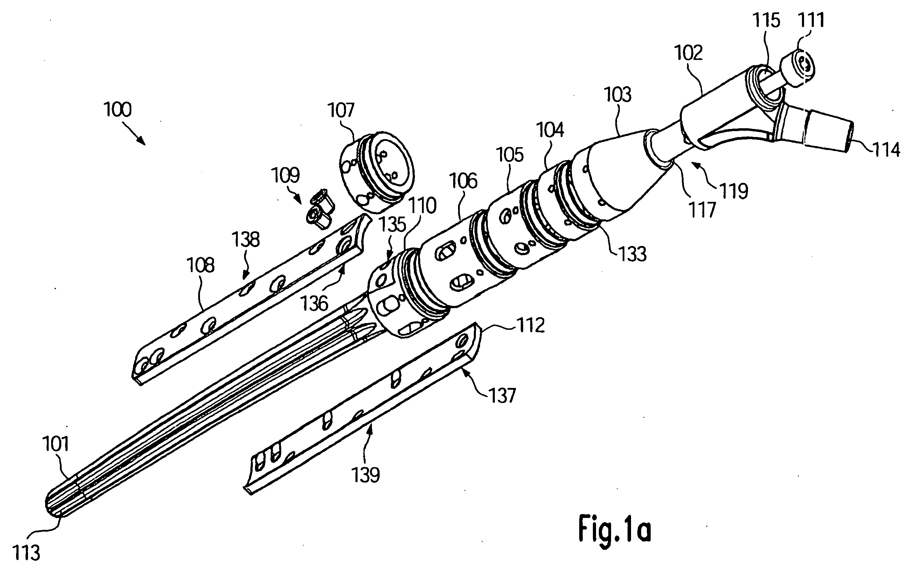

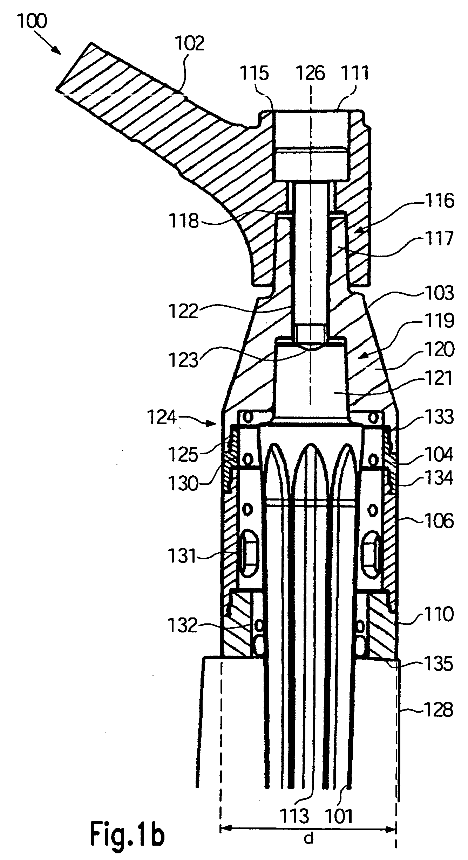

Fig. 1 a eine schematische Perspektivansicht eines modularen Hüftimplantats gemäß einer Ausführungsform der vorliegenden Erfindung;
Fig. 1 b eine schematische Querschnittsansicht des in Fig. 1 a gezeigten Hüftimplantats in implantiertem Zustand; und
Fig. 2a und 2b schematische Skizzen von Zwischenstücken, die gemäß der vorliegenden Erfindung in einem Hüftimplantat verwendet werden können.
Fig. 1 a zeigt eine schematische Perspektivansicht eines modularen Hüftimplantats 100 gemäß einer Ausführungsform der vorliegenden Erfindung. Eine schematische Querschnittsansicht des Hüftimplantats 100 in implantiertem Zustand ist in Fig. 1 b gezeigt.

Das Hüftimplantat 100 umfasst einen Schaft 101, ein Halsteil 102, ein Zwischenstück 103 und Hülsenelemente 104, 105, 106, 107, 110. Ferner sind Platten 108, 112 vorgesehen, die durch Schrauben 109 an einem oder mehreren der Hülsenelemente 104, 105, 106, 107, 110 befestigt werden können und die dafür ausgelegt sind, mit einem Femur verbunden zu werden.

Der Schaft 101 ist dafür ausgelegt, in einen Femur eines Patienten eingesetzt zu werden. Der Schaft 101 kann in Ausführungsformen der Erfindung ein Schaft eines den Fachleuten bekannten Typs sein, der auch in einem Hüftimplantats nach dem Stand der Technik verwendet werden kann. In manchen Ausführungsformen der vorliegenden Erfindung kann der Schaft 101 für eine zementfreie Implantation ausgelegt sein. Zu diesem Zweck kann der Schaft 101 an einer Außenseite Rillen 113 aufweisen, die entlang einer Längsrichtung des Schafts 101 verlaufen. In anderen Ausführungsformen kann der Schaft 101 dafür ausgelegt sein, in den Femur des Patienten einzementiert zu werden. In solchen Ausführungsformen können die Rillen 113 weggelassen werden.

Das Halsteil 102 weist ein Verbindungselement 114 auf, durch welches das Halsteil 102 mit einer Gelenkkugel (nicht gezeigt) eines den Fachleuten bekannten Typs verbunden werden kann. Das Verbindungselement 114 kann beispielsweise einen männlichen Konus umfassen, auf den die Gelenkkugel gesteckt werden kann.

Das Halsteil 102 umfasst ferner eine durchgehende Längsbohrung 115, durch die eine Schraube 111 geführt werden kann, um das Halsteil 102 mit dem Schaft 101 zu verbinden. In Ausführungsformen der vorliegenden Erfindung kann das Halsteil 102 ein Halsteil eines den Fachleuten bekannten Typs sein, das auch in einem Hüftimplantat nach dem Stand der Technik verwendet werden kann.

Das Zwischenstück 103 ist mit dem Schaft 101 und dem Halsteil 102 verbindbar. Zu diesem Zweck weist das Zwischenstück 103 ein erstes Verbindungselement 116 auf, das zum Verbinden des Zwischenstücks 103 mit dem Halsteil 102 ausgelegt ist. Das erste Verbindungselement kann in Form eines männlichen Konus 117 bereitgestellt werden, der dafür ausgelegt ist, in einen am Halsteil 102 vorgesehenen weiblichen Konus 118 eingeführt zu werden.

Das Zwischenstück 103 weist ferner ein zweites Verbindungselement 119 auf, das zum Verbinden des Zwischenstücks 103 mit dem Schaft 101 ausgebildet ist. Das zweite Verbindungselement 119 kann in Form eines weiblichen Konus 120 bereitgestellt werden, der dafür ausgelegt ist, einen am Schaft 101 angebrachten männlichen Konus 121 aufzunehmen. In anderen Ausführungsformen der Erfindung kann das zweite Verbindungselement 119 einen männlichen Konus umfassen, der dafür ausgelegt ist, in einen am Schaft 101 angebrachten weiblichen Konus eingesetzt zu werden.

Um das Zwischenstück 103 mit dem Halsteil 102 und dem Schaft 101 zu verbinden, wird das erste Verbindungselement 116 in das Halsteil 102 gesteckt und der Schaft 101 in das zweite Verbindungselement 119 gesteckt. Anschließend wird eine Schraube 111 durch die Bohrung 115 im Halsteil und eine im Zwischenstück 103 vorgesehene durchgehende Längsbohrung 122 geführt und in ein im Schaft 101 vorgesehenes Gewinde 123 geschraubt. Dadurch werden das Zwischenstück 103, das Halsteil 102 und der Schaft 101 aneinander befestigt.

Das Zwischenstück 103 weist ferner ein drittes Verbindungselement 124 auf, das dafür ausgelegt ist, das Zwischenstück 103 mit einem der Hülsenelemente 104, 105, 106, 107, 110 zu verbinden. Das dritte Verbindungselement 124 kann beispielsweise in Form eines Gewindes 125 bereitgestellt werden, das ringförmig um eine im Zwischenstück 103 vorgesehene Öffnung herum verläuft, durch die der Schaft 101 zum Einführen in das zweite Verbindungselement 119 gesteckt werden kann. Während das Gewinde 125 in manchen Ausführungsformen der vorliegenden Erfindung ein Innengewinde sein kann, das um einen inneren Umfang der Öffnung des Zwischenstücks 103 herum verläuft, kann das Gewinde 125 in anderen Ausführungsformen ein Außengewinde sein, das um einen äußeren Umfang des Zwischenstücks 103 herum verläuft.

In weiteren Ausführungsformen umfasst das dritte Verbindungselement 124 anstelle des Gewindes 125 eine Befestigungsvorrichtung eines anderen Typs, beispielsweise einen Bajonettverschluss oder eine Steckverbindung.

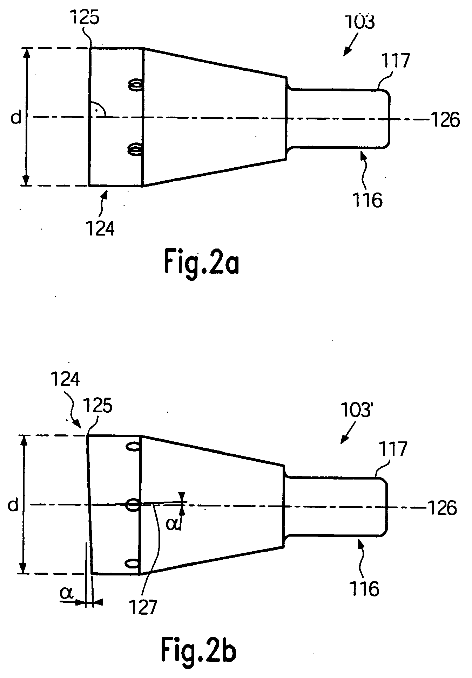

Eine schematische Skizze des Zwischenstücks 103 ist in Fig. 2a gezeigt. Das Zwischenstück 103 kann im wesentlichen eine Rotationssymmetrie bezüglich Drehungen um eine Längsachse 126 aufweisen. Die Längsachse 126 des Zwischenstücks 103 kann dabei durch das erste Verbindungselement 116, das zweite Verbindungselement 119 und die Längsbohrung 122 verlaufen. In Ausführungsformen der Erfindung, in denen das erste Verbindungselement 116 einen männliche Konus 117 und das zweite Verbindungselement 119 einen weibliche Konus 120 umfasst, kann die Längsachse 126 mit einer Achse des männlichen Konus 117 und/oder einer Achse des weiblichen Konus 120 zusammenfallen. Die Längsachse 126 kann ferner mit einer Achse des Gewindes 125 des dritten Verbindungselements 125 zusammenfallen.

In anderen Ausführungsformen der Erfindung kann eine Form des Zwischenstücks 103 von der Rotationssymmetrie abweichen. Fig. 2b zeigt ein Zwischenstück 103' gemäß einer derartigen Ausführungsform der vorliegenden Erfindung. Das Zwischenstück 103' weist eine Längsachse 126 auf, die mit einer Achse eine männlichen Konus 117 eines ersten Verbindungselements 116 zusammenfällt. Im Inneren des Zwischenstücks 103 ist ein (in der Ansicht der Fig. 2b nicht sichtbares) zweites Verbindungselement ähnlich dem zweiten Verbindungselement 119 des Zwischenstücks 103 vorgesehen, das einen weiblichen Konus umfasst, dessen Achse mit der Längsachse 126 zusammenfällt. Das Zwischenstück 103' weist ferner ein drittes Verbindungselement 124 auf, das zum Herstellen einer Verbindung mit einem der Hülsenelemente 104, 105, 106, 107, 110 ausgebildet ist und ein Gewinde 125 umfassen kann. In der in Fig. 2b gezeigten Ausführungsform fällt eine Achse 127 des Gewindes 125 nicht mit der Längsachse 126 zusammen, sondern ist um einen Winkel α gegen diese geneigt. Wie unten genauer ausgeführt wird, ermöglicht dies, eines der Hülsenelemente 104, 105, 106, 107, 110 schräg an das Zwischenstück 103 anzusetzen. Der Winkel α kann einen Wert im Bereich von 0,5 bis 6° haben. In einer Ausführugsform der vorliegenden Erfindung kann der Winkel α einen Wert von 2.5° haben.

Die Hülsenelemente 104, 105, 106, 107, 110 können eine im wesentlichen zylindrische Form aufweisen, wobei ein Außendurchmesser der Hülsenelemente 104, 105, 106, 107, 110 im wesentlichen gleich einem Außendurchmesser d des Zwischenstücks 103, 103' an dessen dem Schaft 101 zugewandtem Ende, an dem sich das zweite Verbindungselement 119 befindet, ist. Der Außendurchmesser d des Zwischenstücks bzw. der Außendurchmesser der Hülsenelemente 104, 105, 106, 107, 110 können dabei in etwa einem Durchmesser eines menschlichen Femur 128 (Fig. 1b) entsprechen. Insbesondere können der Außendurchmesser d des Zwischenstücks 103 und die Außendurchmesser der Hülsenelemente 104, 105, 106, 107, 110 Werte in einem Bereich von 20 bis 50 mm; insbesondere 20 bis 35 mm; oder bevorzugt 22 bis 30 mm haben. In einer Ausführungsform können die Außendurchmesser einen Wert von 30 mm haben.

Jedes der Hülsenelemente 104, 105, 106, 107, 110 weist eine durchgehende Längsbohrung auf. In Fig. 1 b bezeichnet das Bezugszeichen 130 eine Längsbohrung des Hülsenelements 104, das Bezugszeichen 131 eine Längsbohrung des Hülsenelements 106 und das Bezugszeichen 132 eine Längsbohrung des Hülsenelements 110. Die Längsbohrungen 130, 131, 132 der Hülsenelemente können eine zylindrische Form haben, wobei ein Innendurchmesser der Längsbohrungen 130, 131, 132 derart ausgelegt ist, dass der Schaft 101 durch die Längsbohrungen 130, 131, 132 hindurch gesteckt werden kann. Insbesondere ist ein Innendurchmesser der Längsbohrungen 130, 131, 132 größer als ein Durchmesser des Schafts 101. Der Durchmesser der Längsbohrungen der Hülsenelemente kann dabei größer als ein Durchmesser der Längsbohrung 122 des Zwischenstücks 103 sein.

Die Hülsenelemente 104, 105, 106, 107, 110 weisen ferner Verbindungselemente auf, mittels derer die Hülsenelemente 104, 105, 106, 107, 110 miteinander und/oder mit dem Zwischenstück 103 verbindbar sind. Beispielsweise weist das Hülsenelement 104 ein Gewinde 133 auf, das als Gegengewinde zu dem Gewinde 125 des dritten Verbindungselements 124 des Zwischenstücks 103 ausgebildet ist. Somit kann das Hülsenelement 104 mit dem Zwischenstück 103 verschraubt werden und dadurch mit diesem verbunden werden. An einem dem Gewinde 133 gegenüberliegenden Ende weist das Hülsenelement 104 ein zweites Gewinde 134 auf, das dem Gewinde 125 des Zwischenstücks 103 entspricht.

Von den anderen Hülsenelementen 105, 106, 107, 110 weisen die Hülsenelemente 105, 106 ebenfalls an beiden Enden Verbindungselemente, die als Gewinde ausgebildet sein können, auf, wobei eines der beiden Gewinde als Gegengewinde zu dem Gewinde 125 des Zwischenstücks 103 ausgebildet ist und das andere Gewinde dem Gewinde 125 entspricht. Somit können die Hülsenelemente 104, 105, 106 in beliebiger Reihenfolge und in beliebigen Kombinationen miteinander verbunden werden.

Die Hülsenelemente 107, 110 sind als Abschluss-Hülsenelemente ausgebildet. Sie weisen jeweils an einem ersten Ende ein Verbindungselement auf, das zum Verbinden mit dem dritten Verbindungselement 124 des Zwischenstücks 103 geeignet ist und als Gegengewinde zu dem Gewinde 125 des Zwischenstücks 103 ausgebildet sein kann. An einem zweiten Ende von jedem der Hülsenelemente 107, 110 ist eine Auflagefläche ausgebildet, die zur Auflage auf einem Femur geeignet ist. In Fig. 1 b ist die Auflagefläche des Hülsenelements 110 durch das Bezugszeichen 135 bezeichnet. Die Auflagefläche des Hülsenelements 107 kann ähnlich wie die Auflagefläche 135 des Hülsenelements 110 ausgebildet sein.

In Ausführungsformen der vorliegenden Erfindung kann die Auflagefläche 135 in Form einer im wesentlichen ebenen Querschnittsfläche des zylindrischen Hülsenelements 110 ausgebildet sein. Ein Innendurchmesser der Längsbohrung des Hülsenelements 110 kann kleiner als ein Innendruchmesser der Längsbohrungen der anderen Hülsenelemente 104, 105, 106, 107 sein. Vorteilhafterweise wird dadurch eine größere Fläche bereitgestellt, mit der das Hülsenelement 110 auf dem Femur 128 aufliegen kann.

In anderen Ausführungsformen kann die Auflagefläche 135 zu der Längsachse des Hülsenelements 110 geneigt sein, so dass das Hülsenelement 110 auf einem schräg abgeschnittenen Femur des Patienten aufliegen kann.

In manchen Ausführungsformen kann die Auflagefläche eine Beschichtung aus einem das Knochenwachstum fördernden Stoff aufweisen, beispielsweise eine Beschichtung aus Hydroxylapatit. Dadurch kann eine feste und dauerhafte Verbindung zwischen dem Knochen des Patienten und der Auflagefläche gefördert werden.

Die Verbindungselemente der Hülsenelemente 104, 105, 106, 107, 110 müssen nicht, wie oben beschrieben, in Form von Gewinden bereitgestellt werden. In anderen Ausführungsformen der Erfindung können anstelle von Gewinden Bajonettverschlüsse und/oder Steckverbindungen vorgesehen sein.

Die Hülsenelemente 104, 105, 106, 107, können unterschiediche Längen aufweisen. Durch Verbinden von einem oder mehreren der Hülsenelemente 104, 105, 106, 107 mit dem Zwischenstück 103, 103' kann so eine unterschiedliche Länge einer den Schaft 101 ringförmig umschließenden Hülse, die durch ein oder mehrere mit dem Zwischenstück 103, 103' verbundene Hülsenelemente gebildet wird, bereitgestellt werden.

Wenn eines oder mehrere der Hülsenelemente 104, 105, 106, 107, 110 mit dem Zwischenstück 103 verbunden werden, liegen die Längsbohrungen 130, 131, 132 der Hülsenelemente 104, 105, 106, 107, 110 vor dem zweiten Verbindungselement 119, so dass das zweite Verbindungselement 119 durch die Längsbohrungen 130, 131, 132 zugänglich ist. Der Schaft 101 kann somit durch die Längsbohrungen 130, 131, 132 der Hülsenelemente 104, 105, 106, 107, 110 gesteckt werden, so dass der Schaft 101 in das zweite Verbindungselement 119 eingesetzt werden kann. Wenn der Schaft 101 eingesetzt ist, umschließen die Hülsenelemente 104, 105, 106, 107, 110 den Schaft 101 ringförmig. Ein Abstand zwischen dem distalen Ende des Schafts 101 und dem Halsteil 102 des Hüftimplantats wird dabei von der Länge des Schafts 101 und der Länge des Zwischenstücks 103 bestimmt, nicht aber von der Anzahl und der Länge der verwendeten Hülsenelemente.

Wenn das Hüftimplantat 100 einem Patienten implantiert wird, können eines oder mehrere der Hülsenelemente 104, 105, 106, 107, 110 ausgewählt werden, um einen Zwischenraum zwischen dem noch vorhandenen Stück des Femur des Patienten und dem Halsteil 102 des Hüftimplantats zu füllen. Je nach der Länge des fehlenden Knochenstücks können dabei mehr oder weniger Hülsenelemente oder unterschiedlich lange Hülsenelemente verwendet werden. Auf der distalen Seite kann dabei ein Abschluss-Hülsenelemente angeordnet werden, um einen verbesserten Kontakt zwischen dem Hüftimplantat und dem Knochen des Patienten herzustellen.

Da, wie oben ausgeführt, der Abstand zwischen dem distalen Ende des Schafts 101 und dem Halsteil 102 des Hüftimplantats unabhängig von der Länge und Anzahl der Hülsenelemente ist, muss die Schaftlänge nicht an die Länge und Anzahl der Hülsenelemente angepasst werden. Somit kann auch bei Patienten, denen ein großes Stück des Femur entfernt werden musste, ein Standardschaft verwendet werden.

Viele Patienten haben einen leicht gekrümmten Femur. Bei solchen Patienten kann es vorteilhaft sein, wenn der Schaft 101 ebenfalls eine leichte Krümmung aufweist, da so das Hüftimplantat 100 besser an die Anatomie des Patienten angepasst werden kann.

In Ausführungsformen der vorliegenden Erfindung, in denen ein gekrümmter Schaft verwendet wird, kann es vorteilhaft sein, wenn das oben beschriebene Zwischenstück 103' verwendet wird, bei dem eine Achse des Gewindes 125 des dritten Verbindungselements 124 gegen die Längsachse des Zwischenelements 103' geneigt ist. Durch die Neigung des Gewindes können eines oder mehrere der Hülsenelemente 104, 105, 106, 107, 110 leicht schräg an das Zwischenstück 103' angesetzt werden, so dass eine bessere Abstimmung zwischen der Richtung der Längsbohrungen der Hülsenelemente 104, 105, 106, 107, 110 und der Richtung des Schafts 101 erreicht werden kann.

In manchen Ausführungsformen der Erfindung, in denen der Schaft 101 eine Krümmung aufweist, können auch die Achsen der beiden Gewinde eines Hülsenelements.

104, 105, 106 gegeneinander geneigt sein. Ein Winkel, um den die Gewinde gegeneinander geneigt sind, kann dabei einen Wert in einem Bereich von 0,5° bis 6°, insbesondere einen Wert von 2,5° haben. Dadurch sind im zusammengebauten Hüftimplantat 100 die Längsbohrungen der einzelnen Hülsenelemente leicht gegeneinander geneigt, so dass ein durch die Längsbohrungen gebildeter Kanal der Krümmung des Schafts angepasst sein kann.

In Ausführungsformen der vorliegenden Erfindung, in denen ein gerader Schaft 101 verwendet wird, kann das Zwischenstück 103 verwendet werden, in dem eine Achse des Gewindes 125 des dritten Verbindungselements 124 mit der Längsachse des Zwischenstücks 103 zusammenfällt. Dadurch kann der gerade Schaft 101 in Richtung der Längsbohrung eines mit dem Zwischenstück 103 verbundenen Hülsenelements in das zweite Verbindungselement 119 eingesetzt werden. Ferner können in Ausführungsformen der vorliegenden Erfindung mit geradem Schaft 101 die Achsen der Gewinde der Hülsenelemente 104, 105, 106, 107, 110 mit den Längsachsen der Längsbohrungen der Hülsenelemente 104, 105, 106, 107, 110 zusammenfallen, so dass im zusammengebauten Hüftimplantat 100 die Längsbohrungen der Hülsenelemente 104, 105, 106, 107, 110 in die gleiche Richtung verlaufen und einen geraden Kanal bilden.

Jedes der Hülsenelemente 104, 105, 106, 107, 110 kann Öffnungen umfassen, die durch eine Wandung des jeweiligen Hülsenelements verlaufen und einen Innenraum der Längsbohrung des Hülsenelements mit der Umgebung verbinden. Dadurch kann vermieden werden, dass im implantierten Hüftimplantat 100 ein Hohlraum entsteht, in dem sich Infektionskeime ansammeln können. In manchen Ausführungsformen der vorliegenden Erfindung können eines oder mehrere der Hülsenelemente 104, 105, 106, 107, 110 Öffnungen mit unterschiedlichen Durchmessern aufweisen. In manchen. Ausführungsformen können z.B. zusätzlich zu runden Öffnungen auch längliche Öffnungen vorgesehen sein. Die Öffnungen in den Wandungen der Hülsenelemente können auch zum Ansetzen eines Werkzeugs zum Festschrauben der Hülsenelemente 104, 105, 106, 107, 110 verwendet werden.

Das Hüftimplantat 100 kann ferner Platten 108, 112 umfassen, die an einem der Hülsenelemente 104, 105, 106, 107, 110 und/oder dem Zwischenstück 103, 103' befestigbar sind. Beispielsweise kann das Hülsenelement 110 Gewindebohrungen 135 aufweisen, in die Schrauben 109 einschraubbar sind. In den Platten 108, 112 können Öffnungen 136, 137 vorgesehen sein, durch welche die Schrauben 109 geführt werden können. Somit können die Platten 108, 112 an das Hülsenelement 110 angeschraubt werden. Das Hülsenelement 110 kann in der Umgebung der Gewindebohrungen 135 Vertiefungen aufweisen, deren Form an die Form der Platten 108, 112 angepasst ist, so dass proximale Enden der Platten 108, 112 in die Vertiefungen eingelegt werden können. Dadurch kann eine Stabilität der Verbindung zwischen den Platten 108, 112 und dem Hülsenelement 110 verbessert werden.

In anderen Ausführungsformen können die Platten 108, 112 zusätzlich oder alternativ zur Befestigung am Hülsenelement 110 an anderen Hülsenelementen 104, 105, 106, 107 und/oder am Zwischenstück 103, 103' befestigbar sein.

Jede der Platten 108, 112 weist Öffnungen 138, 139 auf, durch die Knochenschrauben geführt werden können, mit deren Hilfe die Platten 108, 112 mit dem Femur 128 des Patienten verbunden werden können. Dadurch wird neben der Fixierung des Hüftimplantats 100 durch den Schaft 101 eine zusätzliche Befestigungsmöglichkeit des Hüftimplantats 100 am Knochen des Patienten geschaffen, durch die eine Stabilität der Verbindung zwischen dem Hüftimplantat 100 und dem Knochen verbessert werden kann. Eine Verwendung der Platten 108, 112 kann insbesondere bei Patienten mit brüchigen Knochen, z.B. Patienten, die unter Osteoporose und/oder Knochentumoren leiden, von Vorteil sein.

In Ausführungsformen der vorliegenden Erfindung können der Schaft 101 und das Halsteil 102 des Hüftimplantats 100 einem Schaft und einem Halsteil eines Hüftimplantats nach dem Stand der Technik entsprechen. Beispielsweise können der Schaft 101 und das Halsteil 102 im Wesentlichen identisch zu entsprechenden Komponenten in einer Revisionshüftprothese nach dem Stand der Technik sein. Vorteilhafterweise ermöglicht dies, Komponenten, die sich in Hüftimplantaten für Standardoperationen bewährt haben, auch Patienten mit großen Defekten des Femur zur Verfügung zu stellen. Somit können bewährte diaphysäre Verankerungsprinzipien auch bei solchen Patienten abgewendet werden.

In solchen Ausführungsformen kann die vorliegende Erfindung in Form eines Moduls für ein Hüftimplantat bereitgestellt werden, welches das Zwischenstück 103 und/oder das Zwischenstück 103' sowie eines oder mehrere der Hülsenelemente 104, 105, 106, 107, 110 und wahlweise auch die Platten 108, 112 umfasst. Vorteilhafterweise muss in derartigen Ausführungsformen kein vollständiges, speziell auf die Bedürfnisse von Patienten mit großen Defekten im Femur abgestimmtes Implantat vorrätig gehalten werden, um solche Patienten bei Bedarf versorgen zu können. Stattdessen kann unter Verwendung des Moduls ein Hüftimplantat, das für die Versorgung unkomplizierterer Standardfälle ausgelegt ist und deshalb häufig in größerer Stückzahl vorrätig gehalten wird, bei Bedarf umgerüstet werden.

In weiteren Ausführungsformen der vorliegenden Erfindung kann ein Hüftimplantat in Form eines modularen Systems bereitgestellt werden, das - neben dem Schaft 101 und dem Halsteil 102 - sowohl das Zwischenstück 103 und/oder das Zwischenstück 103', die Hülsenelemente 104, 105, 106, 107, 110 und optional die Platten 108, 112 umfasst als auch einen zusätzlichen Verbindungsabschnitt, der nicht zur Verwendung mit den Hülsenelementen 104, 105, 106, 107, 110 und/oder den Platten 108, 112 ausgerüstet ist. Der zusätzliche Verbindungsabschnitt kann beispielweise, ähnlich wie in dem in der DE 43 20 086 A1 beschriebenen Hüftimplantat, Hülsensegmente umfassen, die zwischen den Schaft 101 und das Halsteil 102 eingesetzt werden können.

Die einzelnen Komponenten des modularen Systems können je nach Bedarf miteinander kombiniert werden, um einen Knochendefekt eines Patienten versorgen zu können. Insbesondere können das Halsteil 102 und der Schaft 101 sowohl in das erste 116 bzw. das zweite 119 Verbindungselement der Zwischenstücke 103, 103' als auch in Verbindungselemente des zusätzlichen Verbindungsabschnitts eingesetzt werden. Als zusätzliche Option kann der männliche Konus 121 des Schafts 101 direkt in den weiblichen Konus 118 des Halsteils 102 eingesetzt werden, um ein besonders kurzes Implantat zu schaffen.

In manchen Fällen kann die Entscheidung, eines der Zwischenstücke 103, 103', den zusätzlichen Verbindungsabschnitt oder gar kein Zwischenstück zu verwenden, erst während der Operation getroffen werden. Somit ermöglich das modulare System dem Operateur, das Hüftimplantat flexibel an die im Körper des Patienten vorgefundene Situation anzupassen. Insbesondere kann bei zementfreier Implantation des Schafts 101 in den Femur des Patienten eine Einschublänge des Schafts nicht exakt vorhersehbar sein. Bei Hüftgelenks-Revisionen, bei denen einem Patienten ein bereits vorhandenes künstliches Hüftgelenk ausgetauscht wird, kann es je nach dem Zustand des Femur in der Umgebung des alten Implantats notwendig sein, ein mehr oder weniger großes Stück des Femur zu entfernen. Der modulare Aufbau des Systems ermöglicht es dem Operateur, das Hüftimplantat an die sich ergebende Situation anzupassen. In manchen Ausführungsformen der vorliegenden Erfindung kann beispielsweise eines der Zwischenstücke 103, 103' in Kombination mit einem oder mehreren der Hülsenelemente 104, 105, 106, 107, 110 bei Resektionslängen von 100 mm oder mehr verwendet werden, während bei kleineren Resektionslängen der zusätzliche Verbindungsabschnitt verwendet wird oder, alternativ, der Schaft 101 direkt in das Halsteil 102 eingesetzt wird.

In manchen Ausführungsformen der vorliegenden Erfindung kann ein Hüftimplantat anstelle eines Zwischenstücks 103, 103' ein besonders ausgebildetes Halsteil umfassen, das neben einem ersten Verbindungselement, in das der Schaft 101 einsetzbar ist, ein zweites Verbindungselement aufweist, mittels dessen die Hülsenelemente 104, 105, 106, 107, 110 mit dem Halsteil verbindbar sind. Das zweite Verbindungselement kann dabei Merkmale entsprechend denen des oben beschriebenen dritten Verbindungselements 124 des Zwischenstücks 103 bzw. des Zwischenstücks 103' aufweisen. Durch die Einsetzbarkeit des Schafts 101 in das Halsteil kann im Vergleich zu Ausführungsformen der vorliegenden Erfindung, in denen zwischen dem Halsteil und dem Schaft ein Zwischenstück angebracht wird, eine Baulänge des Hüftimplantats verringert werden. Dies kann von Vorteil sein, wenn Patienten mit kleiner Körpergröße mit einem Hüftimplantat versorgt werden müssen.

In manchen Ausführungsformen der vorliegenden Erfindung können einige oder alle Teile des Hüftimplantats aus Titan oder einer Titanlegierung, z.B. TiAI6V4 oder TiAI6Nb7 gefertigt sein. In anderen Ausführungsformen können andere Materialien verwendet werden.

In allen Ausführungsformen der vorliegenden Erfindung können einzelne oder alle Teile des Hüftimplantats einer Bestrahlung mit Stahl- und/oder Glaskugeln unterzogen werden. Wie in der Druckschrift DE 195 17 275 A1 genauer beschrieben, kann die mechanische Haltbarkeit einer Prothese aus Titan oder einer Titanlegierung durch Bestrahlen mit Stahlkugeln verbessert werden.

## Patentansprüche

1. Hüftimplantat (100), umfassend:
einen Schaft (101), der in einen Femur einsetzbar ist;
ein Halsteil (102), das mit einer Gelenkkugel verbindbar ist, wobei das Halsteil eine durch gehende Längsbohrung (115), aufweist;
ein Zwischenstück (103), das mit dem Schaft und dem Halsteil verbindbar ist, wobei das Zwischenstück eine durchgebende Längsbohrung (122) aufweist; **gekennzeichnet**
eine Schraube (111), wobei die Längsbohrungen des Halsteils und des Zwischenstücks dafür ausgelegt sind, die Schraube durchzustecken und in ein im Schaft vorgesehenes Gewinde (123) zu schrauben, um das Halsteil mit dem Schaft zu verschrauben; und
mindestens ein Hülsenelement (104, 105, 106, 107, 110), das eine Längsbohrung aufweist;
dadurch , dass das Hülsenelement derart mit dem Zwischenstück verbindbar ist, dass der Schaft durch die Längsbohrung des Hülsenelements hindurch in das Zwischenstück einsetzbar ist.

2. Hüftimplantat nach Anspruch 1, wobei der Schaft einen männlichen Konus umfasst und das Zwischenstück einen weiblichen Konus umfasst, wobei der weibliche Konus dafür ausgelegt ist, den männlichen Konus aufzunehmen, und wobei eine Achse des weiblichen Konus eine Längsrichtung des Zwischenstücks definiert.

3. Hüftimplantat nach Anspruch 2, wobei das mindestens eine Hülsenelement derart mit dem Zwischenstück verbindbar ist, dass eine Richtung der Längsbohrung des mindestens einen Hülsenelements parallel zur Längsrichtung des Zwischenstücks ist.

4. Hüftimplantat nach Anspruch 2, wobei das mindestens eine Hülsenelement derart mit dem Zwischenstück verbindbar ist, dass eine Richtung der Längsbohrung des mindestens einen Hülsenelements zur Längsrichtung des Zwischenstücks geneigt ist.

5. Hüftimplantat nach einem der Ansprüche 1 bis 4 mit mehreren Hülsenelementen, wobei die Hülsenelemente derart miteinander und mit dem Zwischenstück verbindbar sind, dass der Schaft durch die Längsbohrungen der mehreren Hülsenelemente hindurch in das Zwischenstück einsetzbar ist.

6. Hüftimplantat nach Anspruch 5, wobei die Hülsenelemente derart miteinander verbindbar sind, dass die Längsbohrungen der Hülsenelemente in die gleiche Richtung verlaufen.

7. Hüftimplantat nach Anspruch 5, wobei die Hülsenelemente derart miteinander verbindbar sind, dass die Längsbohrungen von mindestens zweien der Hülsenelemente zueinander geneigt sind.

8. Hüftimplantat nach einem der Ansprüche 4 und 7, wobei der Schaft eine Krümmung aufweist.

9. Hüftimplantat nach einem der Ansprüche 1 bis 8, wobei das Zwischenstück einen männlichen Konus aufweist, der in einen weiblichen Konus des Halsteils einführbar ist.

10. Hüftimplantat nach einem der Ansprüche 1 bis 9, wobei das Zwischenstück und das mindestens eine Hülsenelement durch Gewinde miteinander verbindbar sind.

11. Hüftimplantat nach einem der Ansprüche 1 bis 10, wobei das mindestens eine Hülsenelement ein Abschluss-Hülsenelement umfasst, das eine zur Auflage auf einem Femur geeignete Auflagefläche aufweist.

12. Hüftimplantat nach einem der Ansprüche 1 bis 11, wobei das Zwischenstück eine Längsbohrung aufweist und das Hüftimplantat eine durch die Längsbohrung führbare Schraube umfasst, die dafür geeignet ist, das Halsteil mit dem Schaft zu verschrauben.

13. Hüftimplantat nach einem der Ansprüche 1 bis 12, wobei das mindestens eine Hülsenelement eine Wandung mit einer oder mehreren Öffnungen aufweist.

14. Hüftimplantat nach einem der Ansprüche 1 bis 13, das zusätzlich mindestens eine Platte (108, 112) umfasst, die an dem mindestens einen Hülsenelement und/oder dem Zwischenstück befestigbar ist und mit einem Femur verbindbar ist.

## Claims

1. Hip implant (100) comprising:
a shaft (101) that can be inserted into a femur:
a neck part (102) that can be connected to a head of a bone, the neck part having a longitudinal through-hole (115);
an intermediate piece (103) that can be connected to the shaft and to the neck part, the intermediate piece having a longitudinal through-hole (122);
a screw (111), the longitudinal holes in the neck part and in the intermediate part being designed for the screw to be inserted therethrough and designed to screw said screw into a thread (123) provided in the shaft, in order to screw the neck part to the shaft; and
at least one sleeve element (104, 105, 106, 107, 110) having a longitudinal hole,
**characterised in that**
the sleeve element can be connected to the intermediate piece in such a manner that the shaft can be inserted into the intermediate piece through the longitudinal hole in the sleeve element.

2. Hip implant according to claim 1, wherein the shaft comprises a male taper and the intermediate piece comprises a female taper, wherein the female taper is designed to receive the male taper, and wherein an axis of the female taper defines a longitudinal direction of the intermediate piece.

3. Hip implant according to claim 2, wherein the at least one sleeve element can be connected to the intermediate piece in such a manner that a direction of the longitudinal hole in the at least one sleeve element is parallel to the longitudinal direction of the intermediate piece.

4. Hip implant according to claim 2, wherein the at least one sleeve element can be connected to the intermediate piece in such a manner that a direction of the longitudinal hole in the at least one sleeve element is inclined towards the longitudinal direction of the intermediate piece.

5. Hip implant according to any of claims 1 to 4, comprising a plurality of sleeve elements, wherein the sleeve elements can be connected to one another and to the intermediate piece in such a manner that the shaft can be inserted into the intermediate piece through the longitudinal holes in the plurality of sleeve elements.

6. Hip implant according to claim 5, wherein the sleeve elements can be connected to one another in such a manner that the longitudinal holes in the sleeve elements extend in the same direction.

7. Hip implant according to claim 5, wherein the sleeve elements can be connected to one another in such a manner that the longitudinal holes in at least two of the sleeve elements are inclined with respect to one another.

8. Hip implant according to any of claims 4 to 7, wherein the shaft has a curvature.

9. Hip implant according to any of claims 1 to 8, wherein the intermediate piece comprises a male taper that can be introduced into a female taper of the neck part.

10. Hip implant according to any of claims 1 to 9, wherein the intermediate piece and the at least one sleeve element can be connected to one another by means of a thread.

11. Hip implant according to any of claims 1 to 10, wherein the at least one sleeve element comprises a terminal sleeve element that has a bearing surface which is suitable for resting on a femur.

12. Hip implant according to any of claims 1 to 11, wherein the intermediate piece has a longitudinal hole and the hip implant has a screw that can be guided through the longitudinal hole and is suitable for screwing the neck part to the shaft.

13. Hip implant according to any of claims 1 to 12, wherein the at least one sleeve element comprises a wall having one or more openings.

14. Hip implant according to any of claims 1 to 13, further comprising at least one plate (108, 112) that can be fastened to the at least one sleeve element and/or to the intermediate piece and can be connected to a femur.

## Revendications

1. Implant de hanche (100) comprenant :
un corps allongé (101), qui peut être inséré dans un fémur ;
une partie de col (102), qui peut être reliée à une tête ou rotule d'articulation, la partie de col présentant un alésage longitudinal (115) traversant ;
une pièce intermédiaire (103), qui peut être reliée au corps allongé et à la partie de col, la pièce intermédiaire présentant un alésage longitudinal (122) traversant ;
une vis (111), les alésages longitudinaux de la partie de col et de la pièce intermédiaire étant conçus pour permettre d'y emmancher la vis et la visser dans un taraudage (123) prévu dans le corps allongé, en vue d'assembler la partie de col avec le corps allongé, par vissage ; et
au moins un élément de douille (104, 105, 106, 107, 110), qui présente un alésage longitudinal ;
**caractérisé**
**en ce que** l'élément de douille peut être relié à la pièce intermédiaire de manière telle, que le corps allongé puisse être inséré la pièce intermédiaire, à travers l'alésage longitudinal de l'élément de douille.

2. Implant de hanche selon la revendication 1, dans lequel le corps allongé comprend un cône mâle et la pièce intermédiaire un cône femelle, le cône femelle étant conçu accueillir le cône mâle, et un axe du cône femelle définissant une direction longitudinale de la pièce intermédiaire.

3. Implant de hanche selon la revendication 2, dans lequel ledit au moins un élément de douille peut être relié à la pièce intermédiaire de manière telle, qu'une direction de l'alésage longitudinal dudit au moins un élément de douille soit parallèle à la direction longitudinale de la pièce intermédiaire.

4. Implant de hanche selon la revendication 2, dans lequel ledit au moins un élément de douille peut être relié à la pièce intermédiaire de manière telle, qu'une direction de l'alésage longitudinal dudit au moins un élément de douille soit inclinée par rapport à la direction longitudinale de la pièce intermédiaire.

5. Implant de hanche selon l'une des revendications 1 à 4, comprenant plusieurs éléments de douille, et dans lequel les éléments de douille peuvent être reliés mutuellement et avec la pièce intermédiaire de manière telle, que le corps allongé puisse être inséré dans la pièce intermédiaire à travers les alésages longitudinaux des plusieurs éléments de douille.

6. Implant de hanche selon la revendication 5, dans lequel les éléments de douille peuvent être reliés les uns aux autres de manière telle, que les alésages longitudinaux des éléments de douille s'étendent dans la même direction.

7. Implant de hanche selon la revendication 5, dans lequel les éléments de douille peuvent être reliés les uns aux autres de manière telle, que les alésages longitudinaux d'au moins deux des éléments de douille soient inclinés les uns par rapport aux autres.

8. Implant de hanche selon l'une des revendications 4 et 7, dans lequel le corps allongé présente une courbure.

9. Implant de hanche selon l'une des revendications 1 à 8, dans lequel la pièce intermédiaire présente un cône mâle, qui peut être inséré dans un cône femelle de la partie de col.

10. Implant de hanche selon l'une des revendications 1 à 9, dans lequel la pièce intermédiaire et ledit au moins un élément de douille peuvent être reliés par des filetages.

11. Implant de hanche selon l'une des revendications 1 à 10, dans lequel ledit au moins un élément de douille comprend un élément de douille terminal, qui présente une surface d'appui adaptée à venir en appui sur un fémur.

12. Implant de hanche selon l'une des revendications 1 à 11, dans lequel la pièce intermédiaire présente un alésage longitudinal, et l'implant de hanche comprend une vis qui est adaptée à assurer la liaison par vis de la partie de col avec le corps allongé.

13. Implant de hanche selon l'une des revendications 1 à 12, dans lequel ledit au moins un élément de douille présente une paroi avec une ou plusieurs ouvertures.

14. Implant de hanche selon l'une des revendications 1 à 13, qui comprend en supplément au moins une plaque (108, 112), qui peut être fixée au dit au moins un élément de douille et/ou à la pièce intermédiaire, et qui peut être reliée à un fémur.
